# EUROPÄISCHE PATENTSCHRIFT

(11) **EP 0 690 303 B1**
(45) Veröffentlichungstag und Bekanntmachung des Hinweises auf die Patenterteilung: **09.07.2003**
(21) Anmeldenummer: 95109340.0
(22) Anmeldetag: 16.06.1995
(51) Int. Cl.: G01N 1/38, G01N 33/00, G01N 37/00, G01N 30/00

(54) **Verfahren zur Herstellung einer Standardlösung**
Method for manufacturing a standard solution
Méthode pour la fabrication d'une solution standard

(30) Priorität: 30.06.1994 DE 4422884
(43) Veröffentlichungstag der Anmeldung: 03.01.1996
(73) Patentinhaber: GKSS-Forschungszentrum Geesthacht GmbH, 21502 Geesthacht (DE)
(72) Erfinder: Neidhart, Bernd Prof. Dr. Ing., 21502 Geesthacht (DE)
(74) Vertreter: Seemann, Ralph, Dr. Dipl.-Phys.

(56) Entgegenhaltungen:
- EP-A- 0 051 213
- EP-A- 0 266 216
- EP-A- 0 651 306

## Beschreibung

Die Erfindung betrifft feststoffverdünnte Standards zur Herstellung von Lösungen mit definierten Analytkonzentrationen sowie ein Verfahren zur Herstellung einer gebrauchsfertigen Standardlösung.

Die Bestimmung von Analytkonzentrationen erfolgt auf der Basis von Standards, d.h. die Standardisierung oder Kalibrierung ist ein unverzichtbarer Bestandteil einer analytischen Gehaltsbestimmung. Der Standard liegt in der Regel als Standard- bzw. Kalibrierlösung vor. Die Anforderungen, die an eine Standardsubstanz oder Standardlösung gestellt werden, sind z.B. höchste chemische Reinheit, bekannte stöchiometrische Zusammensetzung, Homogenität, Stabilität im Hinblick auf Zeit, Temperatur, Druck usw., leichte Verfügbarkeit, problemlose und sichere Handhabung, geringer Aufwand bei der Herstellung, niedrige Kosten bei der Anschaffung und Lagerung. Die Standardisierung ist als ein wichtiger Aspekt der Validierung eines Analysenverfahrens Bestandteil aller modernen Qualitätssicherungskonzepte in der analytisch-chemischen Meßtechnik.

Standardlösungen mit bekannter Analytkonzentration werden zur Zeit von den Anwendern entweder als fertige Lösungen gekauft oder aus Standardsubstanzen durch Einwiegen, Auflösen und Auffüllen auf ein definiertes Endvolumen hergestellt. Aus Gründen der Stabilität enthalten kommerzielle Standardlösungen meist relativ hohe Analytkonzentrationen (z.B. 1 g/l). Bei der Herstellung von Standardlösungen in den Anwenderiaboratorien werden ebenfalls vergleichbare Konzentrationen angestrebt und so in Form von sogenannten Stammlösungen die Langzeitstabilität im Sinne einer konstanten Analytkonzentration gewährleistet. Standardlösungen mit niedrigeren Analytkonzentrationen, z.B. für die Spuren- und Ultraspurenanalyse, werden bei Bedarf aus den Stammlösungen durch Verdünnungsreihen hergestellt. Dabei gilt der analytische Grundsatz, daß mit jedem einzelnen Verdünnungsschritt die Analytkonzentration nur im Verhältnis 1:100 (in Ausnahmefällen 1:1000) reduziert werden darf. Diese Vorgehensweise hat eine Reihe von Nachteilen: das Ausführen eines Pipettierschemas zur Verdünnung birgt zusätzliche Fehlerquellen; Stammlösungen, die meist in 500-ml- bzw. 1000-ml-Gefäßen aufbewahrt werden, benötigen Stand- bzw. Lagerfläche; Verdünnungsreihen erfordern einen zusätzlichen Aufwand an Zeit, Geräten, Verbrauchsmaterial usw.; Verdünnungsreihen sind stets Bestandteil einer Standardverfahrensvorschrift im Rahmen der Qualitätssicherung und können daher kurzfristig nicht modifiziert werden.

Der vorliegenden Erfindung liegt die Aufgabe zugrunde, Standards zur Verfügung zu stellen, bei denen die Verdünnungsschritte entfallen und die genannten Nachteile vermieden werden.

Gegenstand der Erfindung sind feststoffverdünnte Standards zur Herstellung von Lösungen mit definierten Analytkonzentrationen, die dadurch gekennzeichnet sind, daß der Analyt an oder in einem festen Trägermaterial gebunden ist.

Ein weiterer Gegenstand der Erfindung ist ein Verfahren zur Herstellung einer gebrauchsfertigen Standardlösung, das dadurch gekennzeichnet ist, daß eine exakt abgewogene Menge des den Analyten enthaltenden festen Trägermaterials zu einem definierten Volumen an Lösungsmittel gegeben wird.

Bei den erfindungsgemäßen Standards ist der Analyt an oder in einem festen Trägermaterial gebunden, wobei der Massenanteil bzw. Stoffmengenanteil des Trägermaterials deutlich überwiegt. Das Trägermaterial muß für die vorgesehene Verwendung der Standardlösung analytisch inert sein, d.h. es darf zu keinen Störungen im Analysenverfahren kommen. Das Masseverhältnis von Trägermaterial zu Analyt beträgt typischerweise 10³:1 bis 10⁶:1.

Die Qualität des Materials des feststoffverdünnten Standards muß der einer üblichen Standardsubstanz entsprechen. Hinzu kommt die Forderung nach mechanischer Stabilität, d.h. Analyt und Trägermaterial dürfen sich nicht entmischen. Vorzugsweise handelt es sich dabei um ein rieselfähiges Material mit feiner Körnung.

Bei der Belegung von Trägermaterialien ist typischerweise von Korngrößen im Bereichvon 1 µm bis 100 µm auszugehen. Bei Fixierung des Analyten im Trägermaterial durch beispielsweise Isomorphie oder Okklusion sollte der Korngrößenbereich zwischen 0,1 µm und 1 µm liegen.

Durch die Verwendung von relativ großen Mengen an Trägermaterial, an das relativ geringe Mengen an Analyt fixiert sind, ist es möglich, wägbare Mengen (z.B. 0,5 g) von feststoffverdünntem Standard zu erhalten, der den Analyt nur zu einem geringen Anteil enthält. Auf diese Weise ist es möglich, Standardlösungen mit konstanten und sehr kleinen Konzentrationen, z.B. im Bereich von mg bis µg/l, herzustellen.

Geeignete Trägermaterialien für die erfindungsgemäßen feststoffverdünnten Standards sind z.B. Kieselgel, Aluminiumoxid, Reversed-Phase-Material, Ionenaustauscherharz, Cellulose, Alginat, Aktivkohle, Zeolithe, Polymerharze oder eine chemische Reinsubstanz wie Zucker oder Kochsalz.

Die Herstellung der feststoffverdünnten Standards erfolgt durch die Fixierung des Analyten an oder in dem Trägermaterial durch Isomorphie, Okklusion, Clathratbildung, Sorption, Verteilung, lonenaustausch oder Gel-Entrapment. Der Analyt kann bei Raumtemperatur fest, flüssig oder auch gasförmig sein.

Feststoffverdünnte Standards nach der Erfindung können z.B. erhalten werden, indem der Analyt an ein schwerlösliches Trägermaterial wie Kieselgel, Aluminiumoxid, lonenaustauscherharz, Cellulose, Aktivkohle, Zeolithe oder Polymerharze sorptiv oder durch Verteilung fixiert wird. An diese Trägermaterialien können viele organische und anorganische Substanzen gebunden werden. Oft genügt es, die beiden zu verbindenden Substanzen in einem geeigneten Lösungsmittel einige Zeit miteinander in Kontakt zu bringen. Anschließend wird das schwerlösliche Trägermaterial, an dem der Analyt fixiert ist, abgetrennt und getrocknet.

Weitere Möglichkeiten, den Analyten an ein Trägermaterial zu binden, sind z.B. die in situ Präzipitation des Analyten in Gegenwart des Trägermaterials, die Aerosolerzeugung durch Versprühen einer Suspension aus Trägermaterial und Analytlösung mit nachfolgender Trocknung des Aerosols im Gasstrom entlang einer Trockenstrecke, die Verwendung einer Sprühkammer und der Einsatz eines Wirbelbettverfahrens.

Die Fixierung des Analyten in einem Trägermaterial erfolgt beispielsweise durch Co-Präzipitation bzw. Co-Kristallisation, wobei isomorphe bzw. nicht-isomorphe Kristalle oder amorphe Pulver entstehen, in denen der Analyt z.B. durch Einschluß (Okklusion) im Trägermaterial verteilt ist. Eine weitere Möglichkeit, den Analyten in einem Trägermaterial zu fixieren, ist das Gel-Entrapment, bei dem das Gel in einer homogenen Lösung des Analyten hergestellt wird.

Bei der Fixierung von Analyten an Trägermaterialien werden diese in der Regel in den später verwendeten Lösungsmitteln zur Herstellung der Standardlösung unlöslich bzw. schwerlöslich sein; bei der Fixierung von Analyten In Trägermaterialien müssen diese in dem später verwendeten Lösungsmittel zur Herstellung der Standardlösung leicht löslich sein.

Zur Herstellung einer Standardlösung wird der erfindungsgemäße feststoffverdünnte Standard abgewogen und zu einem definierten Volumen an Lösungsmittel gegeben. Das Auflösen des Analyten im Lösungsmittel erfolgt entweder physikalisch oder durch eine chemische Reaktion, wobei das Auflösen in der Regel durch Behandlung des fest/flüssig-Gemisches im Ultraschallbad oder durch Schütteln - in Ausnahmefällen auch durch Erwärmen - sichergestellt wird. Beim Lösevorgang bleibt das Trägermaterial entweder als inerter Bodenkörper zurück oder geht mit in Lösung. Im Falle der Gasanalyse wird der Analyt durch ein Gas, gegebenenfalls bei erhöhter Temperatur, aus dem feststoffverdünnten Standard ausgetrieben.

Über die beiden Variablen Masse an feststoffverdünntem Standard und Lösungsmittel-Volumen wird die gewünschte Analytkonzentration in der resultierenden Standardlösung gesteuert. Verdünnungsschritte entfallen, da der Analyt bereits im feststoffverdünnten Standard in verdünnter Form vorliegt. Der feststoffverdünnte Standard kann als Mono- oder Multianalytstandard vorliegen und ist z.B. für anorganische, organische, biochemische, klinisch-chemische Analysen usw. verwendbar.

Die Verwendung der erfindungsgemäßen feststoffverdünnten Standards hat eine Reihe von Vorteilen gegenüber den Stammlösungen nach dem Stand der Technik: die Fehlerquellen reduzieren sich, weil weniger Arbeitsschritte erforderlich sind; Lagerung und Transport sind einfacher, weil die feststoffverdünnten Standards weniger Stellfläche als Stammlösungen benötigen; der Aufwand an Zeit, Geräten und Verbrauchsmaterial bei der Herstellung einer Standardlösung vermindert sich beträchtlich; Feststoffe sind in der Regel stabiler als Flüssigkeiten.

### Beispiel 1

### Sorption (Chromat auf Kieselgel)

2,5 g Kieselgel werden mit 25 ml einer Chromatlösung (0,5-50 mg/l) versetzt und die Suspension eine Stunde geschüttelt. Danach wird im Wasserstrahlvakuum filtriert und mit 5 ml bidestilliertem Wasser nachgewaschen. Der Rückstand wir im Exsikkator über Calciumchlorid getrocknet. 0,5 g des so erhaltenen feststoffverdünnten Standards werden mit 10 ml Salpetersäure (0,1 mol/l) versetzt und 1,5 min geschüttelt. Die Suspension wird anschließend eine Minute zentrifugiert (3000 Upm) und die überstehende Lösung dekantiert.

Die Chromatkonzentrationen der erhaltenen Lösungen betragen je nach Gehalt der im Sorptionsschritt verwendeten Chromatlösung bis 5 µg/l.

### Beispiel 2

### Mischkristallbildung (Cr(III)-dotierter Kalium-/Aluminiumalaun)

Zu 100 ml einer bei 50 °C gesättigten Kalium-/Aluminiumalaunlösung werden 40 bis 200 µl einer Chrom(III)lösung (4,8 g/l) zugegeben. Dazu werden unter Rühren langsam 30 ml Aceton zugetropft, wodurch eine nahezu vollständige Ausfällung der Mischkristalle erfolgt. Die überstehende Lösung wird dekantiert und der Niederschlag bei 50 °C im Trockenschrank getrocknet. 0,5 g des so erhaltenen feststoffverdünnten Standards werden in 10 ml Salpetersäure (0,1 mol/l) gelöst.

Die Chromkonzentrationen der erhaltenen Lösungen betragen je nach Volumen der bei der Ausfällung verwendeten Chromlösung zwischen 0,1 und 1 mg/l.

### Beispiel 3

### In-situ-Präzipitaton (Strontiumchromat auf Kieselgel)

2 g Kieselgel werden mit Chromatlösung (0,5 mg/l) versetzt und die Suspension 30 min gerührt. Anschließend werden langsam 5 ml einer wäßrigen 10%igen Strontiumchloridlösung zugegeben und für weitere 10 min gerührt. Der Niederschlag wird im Wasserstrahlvakuum abfiltriert, mit 10 ml bidestilliertem Wasser gewaschen und getrocknet. 0,5 g des erhaltenen feststoffverdünnten Standards werden mit 10 ml Salpetersäure (0,1 mol/l) 15 min im Ultraschallbad extrahiert.

Der Extrakt weist einen Chromatgehalt von 25,4 µg/l auf.

### Beispiel 4

### Sorption (Sulfat auf Aluminumoxid)

1 g γ-Aluminiumoxid wird mit 50 ml einer wäßrigen Sulfatlösung (0,5-10 mg/l; pH-Wert < 2) versetzt und die Suspension 30 min geschüttelt. Anschließend wird das Aluminiumoxid abgesaugt und getrocknet. 0,1g des so erhaltenen feststoffverdünnten Standards werden mit 10 ml Ammoniaklösung (0,1 mol/l) versetzt und 5 min geschüttelt. Die Suspension wird eine Minute zentrifugiert (3000 Upm) und die überstehende Lösung dekantiert.

Die Sulfatkonzentrationen der erhaltenen Lösungen betragen je nach Gehalt der im Sorptionsschritt verwendeten Sulfatlösung bis 250 µg/l.

## Patentansprüche

1. Verfahren zur Herstellung einer gebrauchsfertigen Standardlösung, bei dem eine exakt abgewogene Menge eines einen Analyten enthaltenden festen Trägermaterials zu einem definierten Volumen an Lösungsmittel gegeben wird.

2. Verfahren nach Anspruch 1, **dadurch gekennzeichnet, daß** sich der Analyt in dem Lösungsmittel vollständig löst.

3. Verfahren nach einem oder beiden der Ansprüche 1 oder 2, **dadurch gekennzeichnet, daß** rieselfähiges und feinkörniges Trägermaterial verwendet wird.

4. Verfahren nach Anspruch 3, **dadurch gekennzeichnet, daß** als Trägermaterial Kieselgel, Aluminiumoxid, Reversed-Phase-Material, Ionenaustauscherharz, Cellulose, Alginat, Aktivkohle, Zeolith oder eine chemische Reinsubstanz verwendet wird.

5. Verfahren nach einem oder mehreren der Ansprüche 1 bis 4, **dadurch gekennzeichnet, daß** die Fixierung des Analyten an oder in das Trägermaterial durch Isomorphie, Okklusion, Clathratbildung, Sorption, Verteilung, Ionenaustausch oder Gel-Entrapment erfolgt.

## Claims

1. Method for preparing a ready-for-use standard solution in which an exactly weighed-out quantity of a solid support material containing an analyte is added to a defined volume of solvent.

2. Method according to Claim 1, **characterized in that** the analyte dissolves completely in the solvent.

3. Method according to one or both of the Claims 1 or 2, **characterized in that** a free-flowing, fine-grain support material is used.

4. Method according to Claim 3, **characterized in that** silica gel, aluminium oxide, reversed-phase material, ion-exchange resin, cellulose, alginate, active charcoal, zeolite or a pure chemical substance is used as the support material.

5. Method according to one or more of the Claims 1 to 4, **characterized in that** the fixation of the analyte onto or into the support material takes place by isomorphism, occlusion, clathrate formation, sorption, dispersion, ion exchange or gel entrapment.

## Revendications

1. Procédé pour la préparation d'une solution standard prête à l'emploi, dans lequel on ajoute une quantité. exactement pesée d'un support solide renfermant un analyte, à un volume défini de solvant.

2. Procédé selon la revendication 1, **caractérisé en ce que** l'analyte se dissout complètement dans le solvant.

3. Procédé selon une ou les deux revendications 1 ou 2, **caractérisé en ce qu'**on utilise une matière de support finement granulée et fluide.

4. Procédé selon la revendication 3, **caractérisé en ce qu'**on utilise comme matière de support le gel de silice, l'oxyde d'aluminium, une matière à phase inverse, une résine échangeuse d'ions, la cellulose, un alginate, le charbon actif, la zéolite ou une substance chimique pure.

5. Procédé selon une ou plusieurs des revendications 1 à 4, **caractérisé en ce que** l'analyte est fixé sur ou dans la matière de support par isomorphie, occlusion, formation de clathrates, sorption, répartition, échange d'ions ou séquestration dans gel.
